# EUROPEAN PATENT APPLICATION

(11) **EP 1 475 366 A1**
(43) Date of publication of application: **10.11.2004**
(21) Application number: 04016428.7
(22) Date of filing: 15.10.2001
(51) Int. Cl.: C07C 227/18

(54) **Process for the preparation of gabapentin**

(30) Priority: 23.10.2000 IT MI20002285
(62) Divisional of application: 01988708.2
(71) Applicant: ZAMBON GROUP S.p.A., 36100 Vicenza (IT)
(72) Inventor: Cannata, Vincenzo, 40037 Sasso Marconi (BO) (IT); Nicoli, Andrea, 36100 Vicenza (IT); Corcella, Francesco, 20015 Parabiago (MI) (IT)
(74) Representative: Longoni, Alessandra

(57) **Abstract**

The present invention relates to a process for the preparation of gabapentin and, more particularly, it relates to a process for the preparation of gabapentin hydrochloride by the Hofmann rearrangement of 1,1-cyclohexane-diacetic acid monoamide.

## Description

This is a divisional application of the patent application published as EP1341749 originally filed as International Application No. PCT/EP01/11867, on October 15, 2001.

The present invention relates to a process for the preparation of gabapentin and, more particularly, it relates to a process for the preparation of gabapentin hydrochloride by the Hofmann rearrangement of 1,1-cyclohexane-diacetic acid monoamide.

Gabapentin, 1-(aminomethyl)-cyclohexaneacetic acid (The Merck Index, XII Ed., page 733, No. 4343), is a known drug with anti-epileptic activity described for the first time by Warner-Lambert Co. in the US patent 4,024,175.

In the literature several processes for the preparation of gabapentin are reported (see for example the US patents 4,024,175, 5,068,413 and 5,091,567).

Substantially all these methods foresee a final step of gabapentin purification that consists in the treatment of an aqueous solution of a gabapentin salt (generally hydrochloride) through a weak basic ionic exchange resin, the complete evaporation of water from the aqueous gabapentin solution eluted from the resin and the crystallization from an alcoholic solvent, generally methanol or methanol/isopropanol or ethanol/ether mixtures.

Several alternative methods to the use of the weak basic ionic exchange resin for the conversion of gabapentin hydrochloride into gabapentin have been described.

In patent application WO 98/28255 (Teva) a process for the preparation of gabapentin from the corresponding hydrochloride is described which comprises the purification of gabapentin hydrochloride from the inorganic salts deriving from the synthesis by (a) solubilization of gabapentin hydrochloride in organic solvents wherein the inorganic salts are insoluble, (b) filtration and (c) optional evaporation of the solvent; the treatment of a gabapentin hydrochloride solution with an amine in a solvent so as to precipitate gabapentin form III and the crystallization to obtain gabapentin form II.

In patent application WO 00/58268 (Bioindustria Laboratorio Italiano Medicinali S.p.A.) the separation of the inorganic salts from gabapentin is carried out by diafiltration.

We have now found an alternative process for preparing gabapentin from 1,1-cyclohexane-diacetic acid monoamide which is particularly suitable for industrial procedures.

Therefore, object of the present invention is a process for the preparation of gabapentin comprising:
a. reacting 1,1-cyclohexanediacetic acid monoamide with sodium hydroxide and sodium hypochlorite, to obtain a first reaction mixture;
b. adding a reducing agent to said first reaction mixture, to obtain a second reaction mixture;
c. adding hydrochloric acid to said second reaction mixture, to obtain a third mixture comprising gabapentin hydrochloride and at least one inorganic salt.
1,1-cyclohexanediacetic acid monoamide can be prepared according to one of the synthetic methods described in the literature such as, for example, by using the process described for the preparation of the lower cyclic homologue 1,1-cyclopentane-diacetic acid monoamide in the US patent 4,024,175, column 5, lines from 49 to 57.

The process object of the invention comprises a first step of preparing as olution of 1,1-cyclohexanediacetic acid monoamide in demineralised water and sodium hydroxide solution.

Said aqueous solution of 1,1-cyclohexanediacetic acid monoamide and sodium hydroxide is preferably formed by adding 1,1-cyclohexanediacetic acid monoamide to an aqueous solution of sodium hydroxide at a temperature of 0°C to 25°C.

Separately, an aqueous solution of sodium hydroxide and sodium hypochlorite is prepared.

Said aqueous solution of sodium hydroxide and sodium hypochlorite is preferably formed by adding sodium hypochlorite to an aqueous solution of sodium hydroxide at a temperature below 25°C.

Then, in order to obtain the first reaction mixture, the reaction of 1,1-cyclohexanediacetic acid monoamide with sodium hydroxide and sodium hypochlorite is carried out by mixing an aqueous solution of 1, 1 -cyclohexanediacetic acid monoamide and sodium hydroxide with the aqueous solution of sodium hydroxide and sodium hypochlorite previously prepared.

Preferably, said mixing is carried out with cooling and more preferably said mixing is carried out while cooling the first reaction mixture to an internal temperature of about -5°C.

Preferably, said mixing is carried out over a time of 2.5 to 4 hours.

Preferably, said mixing is carried out under nitrogen atmosphere.

Furthermore, when the mixing is carried out with cooling, said first reaction mixture is maintained at a temperature of -5°C/+5°C and then the temperature is raised to 20-25°C after the mixing is complete.

At the end of the mixing reaction, the possible excess of oxidant is destroyed, according to known methods, by adding a reducing agent.

Preferably, the reducing agent is sodium metabisulphite.

To this second reaction mixture, a solution of hydrochloric acid, preferably concentrated, is added while keeping the temperature at the room value. Thus, a third reaction mixture comprising gabapentin hydrochloride and at least one inorganic salt is obtained.

The preparation of the free amino acid from the hydrochloride thereof is carried out by a final step of purification.

The purification step is carried out by known methods such as, for example, those methods previously mentioned.

Preferably, the gabapentin hydrochloride purification from the inorganic salts and its conversion in gabapentin is carried out by treatment of an aqueous gabapentin hydrochloride solution through a strong cationic resin and crystallization from organic solvents, preferably alcoholic solvents.

Therefore, another object of the present invention is a process for the preparation of gabapentin further comprising:
d. treating a mixture comprising gabapentin hydrochloride and at least one inorganic salt with a cationic exchange resin.

The process object of the present invention allows to obtain gabapentin form II directly from an aqueous gabapentin hydrochloride solution containing inorganic salts by carrying out the purification and conversion in a single step through the use of the strong cationic resin.

The inorganic salts present in the aqueous gabapentin hydrochloride solution are generally sodium salts, in particular sodium chloride.

The aqueous gabapentin hydrochloride solution used in the purification comes directly from the third reaction mixture used to synthesize gabapentin after usual work-up (extraction and/or crystallization).

The extraction, for example, is carried out according to known methods. Preferably, it proceeds by adding an alcoholic solvent such as n-butanol during acidification step c at about pH 8-9. At the end of the acid addiction at about pH 5 + 0.2 the aqueous phase is separated and further extracted from an alcoholic solvent gradually adding hydrochloric acid to the aqueous phase till pH 2 ± 0.2. The collected alcoholic phases are mixed with water and the obtained biphasic solution is eluted through the cationic resin.

Otherwise, gabapentin hydrochloride is isolated by adding hydrochloric acid during acidification step c of the process of the invention, up to pH 1 ± 0.5 and by seeding gabapentin hydrochloride.

The so obtained solid is dissolved in water and eluted through the cationic exchange resin.

An embodiment of the process object of the present invention is the following.

To a solution comprising demineralised water and a sodium hydroxide solution, 1,1-cyclohexane-di-acetic acid monoamide is added. The obtained solution is added with cooling to a solution of sodium hypochlorite and sodium hydroxide. Then, the temperature is raised slowly to 20-25°C. At the end of the reaction the possible excess of oxidant is destroyed with a reducing agent and a solution of hydrochloric acid is then added to the obtained mixture till pH 5 + 0.2. The extraction from alcoholic solvents gives a biphasic solution comprising gabapentin hydrochloride and at least one inorganic salt ready for the subsequent purification process.

A preferred embodiment of the process object of the present invention is the following.

To a solution comprising demineralised water and a sodium hydroxide solution at a concentration of 30%, 1,1-cyclohexane-di-acetic acid monoamide is added keeping the temperature between 0°C and 25°C. The obtained solution is added with cooling the reaction mixture to an internal temperature of about -5°C to a solution of sodium hypochlorite and sodium hydroxide previously prepared by adding sodium hypochlorite at a concentration of about 13%, while keeping the temperature below 20-25°C, to a sodium hydroxide solution at a concentration of about 30%. Then, the temperature is raised slowly to 20-25°C. At the end of the reaction the possible excess of oxidant is destroyed with sodium metabisulphite and a solution of hydrochloric acid is then added to the obtained mixture till pH 5 ± 0.2 keeping the temperature at the room value. The extraction from n-butanol, gives a biphasic solution comprising gabapentin hydrochloride and at least one inorganic salt ready for the subsequent purification process.

An alternative embodiment of the process object of the present invention is the following.

To a solution comprising demineralised water and a sodium hydroxide solution, 1,1-cyclohexane-diacetic acid monoamide is added. The obtained solution is added with cooling to a solution of sodium hypochlorite and sodium hydroxide. Then, the temperature is raised slowly to 20-25°C. At the end of the reaction the possible excess of oxidant is destroyed with a reducing agent and a solution of hydrochloric acid is then added to the obtained mixture till pH 5 ± 0.2. By further adding hydrochloric acid up to pH 1 ± 0.5 and seeding with gabapentin hydrochloride, the above gabapentin salt is isolated by crystallization. Gabapentin hydrochloride is dissolved in water and it is ready for the subsequent purification process.

A preferred alternative embodiment of the process object of the present invention is the following.

To a solution comprising demineralised water and a sodium hydroxide solution at a concentration of 30%, 1,1-cyclohexane-di-acetic acid monoamide is added keeping the temperature between 0°C and 25°C. The obtained solution is added with cooling the reaction mixture to an internal temperature of about -5°C to a solution of sodium hypochlorite and sodium hydroxide previously prepared by adding sodium hypochlorite at a concentration of 13%, w hile k eeping t he t emperature b elow 2 0-25°C, to a sodium hydroxide solution at a concentration of 30%. Then, the temperature is raised slowly to 20-25°C. At the end of the reaction the possible excess of oxidant is destroyed with sodium metabisulphite and a solution of concentrated hydrochloric acid is then added to the obtained mixture till pH 5 ± 0.2 keeping the temperature at the room value. By further adding concentrated hydrochloric acid up to pH 1 ± 0.5 and seeding with gabapentin hydrochloride, the above gabapentin salt is isolated by crystallization. Gabapentin hydrochloride is dissolved in water and it is ready for the subsequent purification process.

Starting from the aqueous gabapentin hydrochloride solution or the biphasic one, free gabapentin is obtained according to known methods such as, for example, the elution through a strong cationic exchange resin and crystallization from alcoholic solvents described in the international patent application WO 02/34709 of which the present application is a divisional application.

In order to better illustrate the present invention the following examples are now given.

### Example 1

Demineralised water (146 Kg) and sodium hydroxide in about 30% solution (about 140 Kg) were charged in a reactor. Then 1,1-cyclohexanediacetic acid monoamide (190 Kg) was charged portionwise under stirring keeping the temperature between 0 °C a nd 25°C. After keeping under stirring for about 2 hours at 20-25°C a solution was obtained.

In a second reactor sodium hydroxide in about 30% solution (about 140 Kg) and, while keeping the temperature below 20-25°C, 13% sodium hypochlorite (557 Kg) were charged, under vacuum and stirring. The previously prepared solution of 1,1-cyclohexanediacetic acid monoamide was added in about 2.5/4 hours keeping under a light nitrogen flux and cooling at an internal temperature of about -5°C. The mixture was maintained for about 2 hours at -3/+5°C and raised then slowly to 20°C in about 2-3 hours; keeping then at 20-25°C for about 1 hour.

At the end of the reaction and after having destroyed the possible excess of oxidant with sodium metabisulphite, a solution of hydrochloric acid (about 250 Kg) was added up to pH 5±0.2, controlling the foam and the development of carbon dioxide, keeping the temperature at the room value. During the addition of hydrochloric acid, at pH about 8-9, n-butanol (150 Kg) was charged and then the addition was continued.

At the end of the acid addition (pH 5±0.2), the mixture was kept under stirring for about 30 minutes regulating the temperature at about 20°C and left then at rest for about 1 hour. The aqueous phase was separated at the temperature of about 20°C and treated with n-butanol (150 Kg) and with a hydrochloric acid solution (about 35 Kg) up to pH 3.5±0.2. After keeping under stirring for about 30 minutes and controlling again the pH (2±0.2), the mixture was left to rest for about 1 hour and the aqueous phase was separated at the temperature of about 20°C and treated with n-butanol (150 kg) and with a hydrochloric acid solution (about 15 kg) up to pH 2±0.2.

After keeping under stirring for 30' and controlling the pH (2±0.2), the mixture was left to rest for about 1 hour and then the aqueous phase was separated at the temperature of about 20°C.

Water (1000 Kg) was added to the gathered butanolic phases (containing gabapentin, gabapentin hydrochloride and NaCl) and the obtained biphasic solution was eluted through a column containing a strong cationic resin (IMAC HP 1110). At the end of the biphasic solution elution, the column was further eluted with water (about 1500/1800 Kg) up to obtain at the exit Bx≤0.3.

The column was then eluted with an ammonia solution prepared from 28% ammonia (156 Kg) and water (1290 Kg). At the end of the elution with the ammonia solution, the process was continued with water (about 1200/1300 Kg) up to obtain Bx≃0.3.

In this way about 1600 liters of gabapentin ammonia solution were collected, after discarding the dead volume (fractions which do not contain gabapentin).

The ammonia solution was filtered and concentrated by distillation under vacuum with internal temperature below 40°C up to a thick solid residue.

Methanol (95 Kg) was added to the residue in four portions and the mixture was heated with water thermoregulated in jacket at 55-60°C for about 1 hour. Isopropylic alcohol (395Kg) was added to the obtained homogeneous suspension in about 20/30 minutes, with circulation of water thermoregulated at 60-65°C. At the end of the addition, the mixture was kept under stirring for about 30/60 minutes, always with circulating water thermoregulated at about internal temperature 55°C, and then it was cooled first with water and then with saline solution at internal temperature about -5°C. After keeping at this temperature for at least 1 hour, centrifugating and washing with isopropyl alcohol, about 130-140 Kg of wet product were obtained which were dried under vacuum at 50-55°C for about 24 hours obtaining about 120-130 Kg of gabapentin.

### Example 2

Results analogous to example 1 were obtained by isolating gabapentin hydrochloride by crystallization, by treating the reaction mixture, after having destroyed the possible excess of oxidant, with concentrated hydrochloric acid (about 300 Kg) up to pH 1 ± 0.5 and by seeding with gabapentin hydrochloride.

The so obtained solid was dissolved in water (about 100 Kg) and the solution was treated with a strong cationic resin as described in example 1.

## Claims

1. A process for producing gabapentin, comprising:
a. reacting 1,1-cyclohexanediacetic acid monoamide with sodium hydroxide and sodium hypochlorite, to obtain a first reaction mixture;
b. adding a reducing agent to said first reaction mixture, to obtain a second reaction mixture;
c. adding hydrochloric acid to said second reaction mixture, to obtain a third mixture comprising gabapentin hydrochloride and at least one inorganic salt.

2. A process according to claim 1 wherein the reaction of 1,1-cyclohexanediacetic acid monoamide with sodium hydroxide and sodium hypochlorite is carried out by mixing an aqueous solution of 1,1-cyclohexanediacetic acid monoamide and sodium hydroxide with an aqueous solution of sodium hydroxide and sodium hypochlorite.

3. A process according to claim 2 wherein said aqueous solution of 1,1-cyclohexanediacetic acid monoamide and sodium hydroxide is formed by adding 1,1-cyclohexanediacetic acid monoamide to an aqueous solution of sodium hydroxide at a temperature of 0°C to 25°C.

4. A process according to claim 2 wherein said mixing is carried out with cooling.

5. A process according to claim 4 wherein said mixing is carried out while cooling the first reaction mixture to an internal temperature of about -5°C.

6. A process according to claim 4 wherein said first reaction mixture is maintained at a temperature of -5°C/+5°C and then the temperature is raised to 20-25°C after the mixing is complete.

7. A process according to claim 2 wherein said mixing is carried out over a time of 2.5 to 4 hours.

8. A process according to claim 2 wherein said mixing is carried out under nitrogen.

9. A process according to claim 2 wherein said aqueous solution of sodium hydroxide and sodium hypochlorite is formed by adding sodium hypochlorite to an aqueous solution o f sodium hydroxide at a temperature below 25°C.

10. A process according to claim 1 wherein said inorganic salt is NaCl.

11. A process according to claim 1 wherein the reducing agent is sodium metabisulphite.

12. A process according to claim 1 further comprising:
d. treating a mixture comprising gabapentin hydrochloride and at least one inorganic salt with a strong cationic exchange resin.
